**Europäisches Patentamt**

**European Patent Office**

**Office européen des brevets**

(11) Veröffentlichungsnummer: **0 343 380**
**A1**

## EUROPÄISCHE PATENTANMELDUNG

(21) Anmeldenummer: 89107184.7

(22) Anmeldetag: 21.04.89

(51) Int. Cl.⁴: **G01N 33/50 , G01N 15/14**

(30) Priorität: 02.05.88 DE 3814811

(43) Veröffentlichungstag der Anmeldung:
**29.11.89 Patentblatt 89/48**

(84) Benannte Vertragsstaaten:
**BE CH DE FR GB IT LI NL**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Würzburg, Uwe, Dr.**
**Dessauer Strasse 12**
**D-6110 Dieburg(DE)**
Erfinder: **Rieke, Erwin, Dr.**
**Hermannstrasse 12**
**D-6104 Seeheim(DE)**
Erfinder: **Kroll, Werner, Dr.**
**Schwertstrasse 49**
**D-5660 Solingen(DE)**

(54) **Verfahren und Mittel zur Differenzierung und Bestimmung von Leukozyten.**

(57) Die Erfindung betrifft ein Verfahren und Mittel zur Differenzierung und quantitativen Bestimmung von Leukozyten in Vollblut mit Hilfe der Durchflußcytometrie durch Messung von Zellvolumen, Streulicht und Fluoreszenz. Das Verfahren ist dadurch gekennzeichnet, daß man das Vollblut mit einem die Erythrozyten lysierenden und die Leukozyten fixierenden Agens behandelt, mit einem Gemisch von mindestens zwei Fluoreszenzfarbstoffen aus der Thiazol- und Aminoxanthengruppe . versetzt, die gefärbten Zellen mit einer Wellenlänge anregt und die relativen Intensitäten der Fluoreszenzemission bei mindestens zwei verschiedenen Wellenlängenbereichen gleichzeitig mißt und das Vorwärts- und Weitwinkelstreulicht bestimmt.

EP 0 343 380 A1

EP 0 343 380 A1

## Verfahren und Mittel zur Differenzierung und Bestimmung von Leukozyten

Die Erfindung betrifft Verfahren und Mittel zur Differenzierung und quantitativen Bestimmung von Leukozyten im Vollblut sowie die Verwendung des Mittels in der Durchflußcytometrie oder der Mikroskopie.

Die Blutzellenzählung und -differenzierung von Leukozyten ist eine im Klinik- und Praxisbereich häufig angewendete Laboruntersuchung. Bei der Erstellung des Differentialblutbildes werden die absoluten und prozentualen Anzahlen folgender kernhaltiger Blutzellen bestimmt: Lymphozyten, Monozyten, neutrophile, basophile, eosinophile Granulozyten sowie Vorstufen der lymphatischen und myeloischen Reihe.

Es sind zwei verschiedene Verfahren zur Erstellung von Differentialblutbildern bekannt, das manuelle und das automatische Verfahren, wobei das manuelle Verfahren häufiger angewendet wird. Beim manuellen Verfahren wird auf einem Objektträger ein Blutausstrich angefertigt, getrocknet, fixiert und mit Romanowski-Farbstoffen angefärbt. Die Zellen werden dann unter dem Mikroskop betrachtet, klassifiziert und ausgezählt. Die Anzahl differenzierter Zellen beträgt mindestens 100 Zellen.

Der Nachteil des manuellen Verfahrens ist der große Zeitaufwand zum Erstellen eines Differentialblutbildes, der bei bis zu 10 Minuten liegt. Ein weiterer großer Nachteil dieses Verfahrens ist der breite Konfidenzbereich der Verteilung der einzelnen Spezies. Dies trifft vor allem für die weniger häufig vorkommenden Zellen, wie Monozyten, eosinophile und basophile Granulozyten zu. Außerdem läßt sich damit nur die prozentuale Verteilung der einzelnen Zellarten ermitteln. Für die Ermittlung absoluter Werte muß eine gesonderte Zählung aller weißen Zellen durchgeführt werden.

Die in neuerer Zeit angewendeten automatischen Verfahren zur Erstellung von Differentialblutbildern beruhen auf dem Prinzip der Durchflußcytometrie. Aus der EP-OS 121 261 ist ein solches Verfahren zur Differenzierung von Leukozyten bekannt, wobei die Messung des Zellvolumens als Funktion des Weitwinkelstreulichts eine Unterscheidung von Lymphozyten, Monozyten und Granulozyten erlaubt. In diesem Zusammenhang ist auch der Zusatz von zwei Immunofluoreszenzfarbstoffen (Fluoreszeinisothiocyanat und Phycoerythrin) erwähnt, was jedoch nicht zu einer weiteren Differenzierung uber die genannten drei Populationen hinaus beiträgt.

In der EP-OS 161 770 ist ebenfalls ein durchflußcytometrisches Verfahren zur Differenzierung von Leukozyten beschrieben, bei dem die Probe vor der Messung mit einem lysierenden Mittel behandelt wird, das in der Lage ist, die Erythrozyten zu lysieren und die Leukozytenfraktion zu fixieren. Auch in diesen Fall werden nach Zugabe eines fluoreszenzmarkierten Antikörpers das Zellvolumen, die Weitwinkelstreuung und ein oder mehrere Fluoreszenzmessungen herangezogen, um eine Differenzierung in Granulozyten, Monozyten und Lymphozyten zu erhalten. Außer dem Nachteil, daß auch nach diesem Verfahren nur drei Zelltypen differenziert werden können, sind vor der eigentlichen Durchflußcytometrie Inkubationszeiten von insgesamt 40 Minuten und zwei Zentrifugationen erforderlich.

Der Erfindung liegt die Aufgabe zugrunde, ein Verfahren und Mittel zur Differenzierung und quantitativen Bestimmung von Leukozyten im Vollblut zur Verfügung zu stellen, mit dem die Nachteile der bekannten Verfahren vermieden werden können und mit dem eine sichere Differenzierung von fünf Leukozytenpopulationen in kürzester Zeit ohne Zentrifugation und mit hoher Präzision möglich ist.

Gegenstand der Erfindung ist ein Verfahren zur Differenzierung und quantitativen Bestimmung von Leukozyten in Vollblut mit Hilfe der Durchflußcytometrie durch Messung von Zellvolumen, Streulicht und Fluoreszenz, das dadurch gekennzeichnet ist, daß man das Vollblut mit einem die Erythrozyten lysierenden und die Leukozyten fixierenden Agens behandelt, mit einem Gemisch von mindestens zwei Fluoreszenzfarbstoffen aus der Thiazol- und Aminoxanthengruppe versetzt, die gefärbten Zellen mit einer Wellenlänge anregt und die relativen Intensitäten der Fluoreszenzemission bei mindestens zwei verschiedenen Wellenlängenbereichen gleichzeitig mißt und das Vorwärts- und Weitwinkelstreulicht bestimmt.

Ein weiterer Gegenstand der Erfindung ist ein Mittel zur Durchführung dieses Verfahrens, das ein lysierend und fixierend wirkendes Agens und ein Farbreagenz enthält. Das Agens enthält im wesentlichen ein Saponin und einen niederen Aldehyd und das Farbreagenz mindestens je einen Farbstoff aus der Thiazol- und Aminoxanthengruppe. Das Mittel zur Differenzierung und quantitativen Bestimmung von Leukoyzten kann sowohl automatisiert an einem Durchflußcytometer als auch manuell an einem Fluoreszenzmikroskop eingesetzt werden.

Mit dem erfindungsgemäßen Verfahren können die Leukozyten in die Gruppe der Lymphozyten, Monozyten, neutrophilen, eosinophilen und basophilen Granulozyten sowie Vorstufen der myeloischen und lymphozytären Reihe differenziert werden. Neben der Erfassung der Anteile jeder Einzelpopulation der Gesamtzellmenge kann damit auch die absolute Zahl von Zellen pro $\mu$l Blut erfaßt werden.

Das Verfahren basiert auf dem bekannten Meßprinzip der Durchflußcytometrie. Gemessen werden nach Fixierung und Färbung der Leukozyten bei gleichzeitiger Lysierung der roten Blutzellen, die Parameter

2

Vorwärts- und Weitwinkelstreulicht, zwei Fluoreszenzparameter und das Zellvolumen.

Überraschenderweise konnte eine Farbstoffkombination gefunden werden, die es ermöglicht, die Zellen in nur einem, zudem sehr engen Wellenlängenbereich zu bestrahlen und nach Vorauswahl aller Leukozyten über Vorwärts- und Weitwinkelstreulicht, diese über eine Differenzierung in einem zweidimensionalen Histogramm (Fluoreszenz 1 gegen Fluoreszenz 2) leicht in die oben aufgeführten Gruppen zu unterteilen.

Die Farbstoffkombination besteht aus mindestens zwei Fluoreszenzfarbstoffen aus der Thiazol- und Aminoxanthengruppe. Als Thiazolfarbstoffe eignen sich z.B. Thioflavin TCN (C.I. 49005), Thioflavin S, Primulin (C.I. 49000), vorzugsweise Thioflavin TCN. Als Aminoxanthenfarbstoffe eignen sich z.B. Safranilin, Basic Red 8 (C.I. 45150), Basic Red 1 (C.I. 45160), Acid Violet 9 (C.I. 45190), Basic Red 3 (C.I. 45210), Acid Red 52 (C.I. 45100), Pyronin B (C.I. 45010), Pyronin G (C.I. 45005), vorzugsweise Safranilin.

Die Farbstoffe aus beiden Gruppen werden im Verhältnis 1 : 2 bis 2 : 1, vorzugsweise 1 : 1 eingesetzt, wobei die Konzentration des Farbstoffgemisches im Bereich von 0,6-60 μg/l, vorzugsweise 20-40 μg/l, der Lösung liegen sollte. Die Farbstofflösung enthält außerdem vorzugsweise noch 50-100 mM/l eines Puffers und gegebenenfalls 20 bis 100 mM/l Kochsalz. Der pH-Wert wird mit geeigneten Säuren oder Laugen auf einen neutral bis leicht sauren pH-Wert eingestellt, vorzugsweise auf einen pH-Wert von 7,5.

Das auf die Erythrozyten lysierend und auf die Leukozyten fixierend wirkend Agens enthält einen niederen Aldehyd, vorzugsweise Formaldehyd, ein Saponin, vorzugsweise Digitonin und geeignete Puffersubstanzen, vorzugsweise Tris-Puffer als Grundsubstanz des isotonischen Puffers, der einen pH-Bereich von 5-8,5 aufrechterhalten soll. Ähnliche Vorbehandlungsreagenzien sind z.B. aus der EP-OS 161 770 bekannt.

Das Verfahren nach der Erfindung beruht darauf, daß bestimmte Eigenschaften von Blutzellen durch Farbstoffe angefärbt und in Korrelation mit der Zellgröße gemessen werden. Im Falle einer durchflußcytometrischen Messung werden die Zellen nach der Vorbehandlung angefärbt, über eine Probenauftragung in einen Hüllstrom eingeleitet, von diesem zentriert, unter einem Objektiv hergeführt, wobei die Zellen sequentiell das Objektiv passieren, von z.B. Laserlicht beleuchtet und zur Fluoreszenz angeregt. Auf diese Weise gelingt es, in sehr kurzer Zeit eine hohe Anzahl von Zellen zu vermessen. Das durch die Beleuchtung mit Laserstrahlen von einer gefärbten Zelle ausgesandte Fluoreszenzlicht wird in zwei spektrale Anteile aufgeteilt, die dann jeweils von einem Photomultiplier detektiert und in einen entsprechenden Stromfluß umgewandelt wird. Ebenso wird das Weitwinkelstreulicht registriert. Das Vorwärtsstreulicht wird mit Hilfe einer Photodiode quantifiziert. Die so erhaltenen elektrischen Signale werden digital gewandelt und in einen Computer digital abgelegt. Sie werden in ein- und zweiparametrigen Histogrammen angeordnet. Durch Kombination verschiedener dieser vier Parameter gelingt es, gut separierte Cluster von Blutzellpopulationen zu erhalten.

Während in eindimensionalen Histogrammen nur eine sehr geringe Separation der Zellen stattfindet, läßt sich durch Kombination zweier Parameter zu einem korrelierten zweiparametrigen Histogramm eine deutliche Auftrennung der Cluster feststellen. Eine eindeutige Trennung verschiedener Populationen ist damit jedoch nur sehr schwer möglich. In Überlappungsbereichen ist eine Differenzierung in zwei Populationen nur mit großem mathematischem Aufwand zu erreichen und mit einem großen Unsicherheitsfaktor belastet. Weiterhin ist eine Auftrennung aufgrund dieser eindimensionalen Histogramme nur bei maximal drei Populationen (Lymphozyten, Monozyten und Granulozyten) zu gewährleisten.

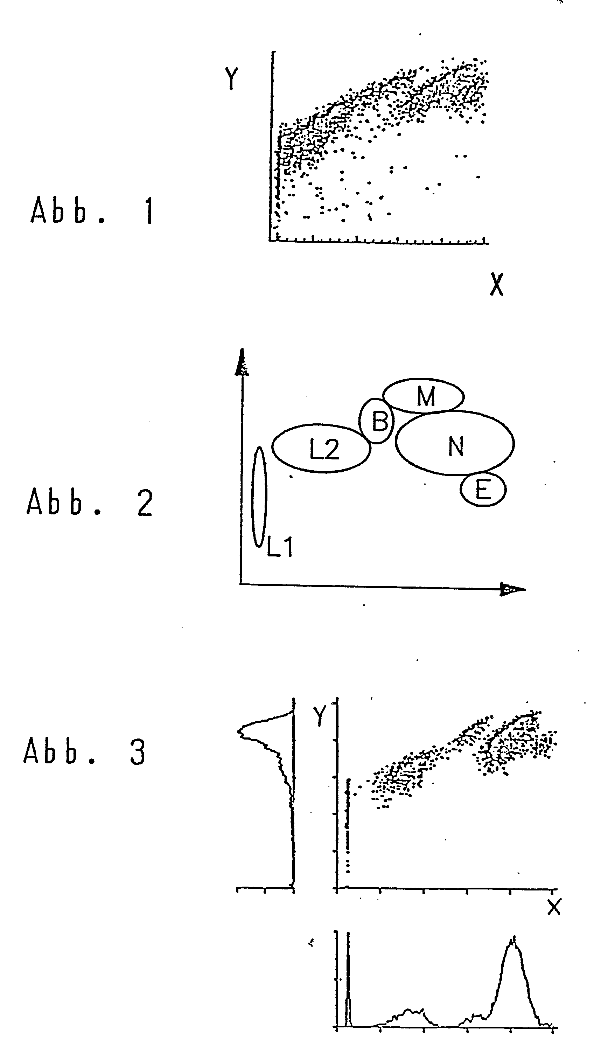

Eine Trennung in fünf Populationen ohne großen mathematischen Aufwand ist erfindungsgemäß erst durch die gleichzeitige und korrelierte Vermessung und Aufnahme von zwei Fluoreszenzemissionen (Grün- und Rotfluoreszenz) ein und derselben Zelle zur gleichen Zeit möglich, wie das Histogramm in Abbildung 1 zeigt. Deutlich sind dort 6 verschiedene Punktwolken zu sehen, die verschiedene Populationen von Leukozyten repräsentieren. Abbildung 1 zeigt ein zweidimensionales Histogramm, in dem die Grünfluoreszenz (y) gegen die Rotfluoreszenz (x) von vorbehandeltem und fluorochromiertem Vollblut aufgetragen ist. In Abbildung 2 ist die Lage der Cluster der verschiedenen Leukozyten im Histogramm nach Abbildung 1 wiedergegeben. Die mit L1 und L2 bezeichneten Populationen repräsentieren die Lymphozyten. Population B repräsentiert die basophilen Granulozyten. Population M die Monozyten. Die neutrophilen Granulozyten sind in Population N und die eosinophilen in Population E wiederzufinden.

Auch ohne großen mathematischen Aufwand ist bereits visuell die Auftrennung in diese 6 Populationen deutlich. Mittels Clusteranalyse läßt sich die Festlegung der Populationen automatisieren. Dadurch ist auch die Verschiebung der Grenzen, wie sie durch individuelle Schwankungen im Blut vorkommen, berücksichtigt. Die Abtrennung kleinerer Blutzellen, wie Erythrozyten und Thrombozyten, erfolgt durch Gaten auf das Vorwärts streulicht, d.h. es wird nur von solchen Zellen die Grün- und Rotfluoreszenz registriert, die eine gewisse Größe im Vorwärtsstreulicht übersteigen. Neben der Differenzierung der Leukozyten erhält man so auch die exakte Zahl dieser Zellen pro Volumeneinheit Probe.

Neben dieser automatisierten Version zur Erstellung eines Differentialblutbildes ist erfindungsgemäß das Färbe- und Vorbehandlungsverfahren auch zur Herstellung eines mikroskopischen Präparates zur Auszählung von Differentialblutbildern geeignet.

Die charakteristischen Merkmale der verschiedenen Leukozyten nach Vorbehandlung und Färbung mit dem erfindungsgemäßen Mittel, lassen sich wie folgt beschreiben:

Neutrophile Granulozyten:

Die ausgereiften neutrophilen Zellen sind in erster Linie spektral identifizierbar. Der Kern fluoresziert intensiv grün, deutlich ist dessen starke Segmentierung zu erkennen. Das Cytoplasma fluoresziert blaß orange. Die Intensität sowohl der Grünals auch der Rotfluoreszenz ist stärker als bei allen anderen Zellen. Ein weiteres Charakteristikum dieser Zellart ist die starke Granulierung des Cytoplasmas mit sehr vielen feinen gelblich fluoreszierenden Körnchen.

Eosinophile Granulozyten:

Diese sind gekennzeichnet durch einen grün fluoreszierenden globulären Nucleus, dessen Rand stark grün zum Cytoplasma hin abgegrenzt ist. Das Cytoplasma leuchtet stark orange und ist völlig homogen. Im Cytoplasma fallen die intensiv gelb fluoreszierenden Granula auf.

Basophile Granulozyten:

Diese besitzen einen kleeblattförmig bis plump segmentierten leuchtend grün fluoreszierenden Kern. Die Grundfarbe des Cytoplasmas ist orange. Vor allem am Zellrand und zwischen den Segmentlücken befinden sich intensiv, sich deutlich vom Cytoplasma abhebende, dunkelorange leuchtende Granula.

Monozyten:

Sie sind charakterisiert durch einen eingebuchteten, gelappten, hellgrün fluoreszierenden Kern. Am Rand fluoresziert der Kern sehr intensiv. Das Plasma fluoresziert im Vergleich zu den Granulozyten schwächer und im Vergleich zu den Lymphozyten stärker gelblich bis orange.

Lymphozyten:

Sie besitzen einen runden bis nierenförmigen Kern, der schwach grün fluoresziert. Mitunter treten Nucleolen im Kern auf. Das Cytoplasma tritt in der Regel nur als schmaler Saum auf, der gelborange fluoresziert.

Das erfindungsgemäße Verfahren wird im allgemeinen wie folgt durchgeführt: Zunächst werden das lysierend und fixierend wirkende Agens (Vorbehandlungslösung) und die Farbreagenzlösung hergestellt. Zur Durchführung des durchflußcytometrischen Verfahrens werden z.B. 50 $\mu$l Blut in einem Reagenzröhrchen vorgelegt und 50 $\mu$l des Vorbehandlungsreagenzes hinzupipettiert. Das Volumenverhältnis von Blut und Vorbehandlungsreagenz sollte zwischen 2 : 1 und 1 : 2 liegen. Nach 5-120 Sekunden Inkubationszeit bei leichter Bewegung der Lösung werden 500 $\mu$l Puffer zu diesem Gemisch hinzugegeben und geschüttelt. Dann werden unter Schütteln 100 $\mu$l der Farbreagenzlösung hinzugegeben. Nach weiteren 5-120 Sekunden Inkubationszeit wird die Lösung gemessen. Dazu wird sie einem Durchflußcytometer zugeführt; die Hüllstromflüssigkeit des Durchflußcytometers besteht aus einem Puffer im pH-Bereich von 7-8. Die Bestrahlung erfolgt bei einer Wellenlänge unterhalb 515 nm und die Fluoreszenzemission wird in den Bereichen von 500-550 und 570-630 nm gemessen.

Zur Durchführung des fluoreszenzmikroskopischen Verfahrens wird ein Tropfen Blut mit einem Tropfen Vorbehandlungsreagenz auf einem Objektträger gemischt. Nach ca. 10 Sekunden Inkubationszeit wird direkt die Farbreagenzlösung hinzugegeben. Das Präparat wird mit einem Deckgläschen eingedeckt und kann dann im Fluoreszenzmikroskop bei einer Anregungswellenlänge < 500 nm und einer Emissionswellenlänge > 500 nm betrachtet werden.

Die zu untersuchenden Blutproben können verschiedenen Quellen entstammen: frische Proben venösen Blutes, angereicherte Leukozyten (Dichtegradienten-Sedimentation) oder Kapillarblut. Als Antikoagulanz kann dem Blut Heparin, EDTA oder Citrat zugesetzt sein; bevorzugt wird Blut mit EDTA als Antigerinnungsmittel verwendet.

Neben den bereits genannten Vorteilen des erfindungsgemäßen Verfahrens (Schnelligkeit, hohe Präzision, Differenzierung von fünf Leukozytenpopulationen) sei noch erwähnt, daß die an einem Durchflußcytometer gewonnenen Daten eine geringe Empfindlichkeit gegen Veränderungen von Reaktionstemperatur und Vorbehandlungs- bzw. Färbezeit aufweisen.

Außerdem ist die Verwendung auch älteren Blutes, d.h. bis zu 24-36 Stunden altem Blut, möglich. Ein weiterer Vorteil ist in der direkten Vergleichbarkeit des Fluoreszenzmusters der Zellen im Fluoreszenzmikroskop mit konventionellen Färbeverfahren zu sehen (z.B. Wright-Stain im Durchlicht), d.h. es lassen sich leicht Vergleiche zu den bisher verwendeten manuellen Verfahren finden.

Beispiel 1

Ein Teil venösen EDTA-Blutes wird mit einem Teil der Vorbehandlungslösung gemischt, die folgende Zusammensetzung aufweist:

| 1. Formalin (37 %) | 200 ml |
|---|---|
| 2. Digitonin | 20 g |
| 3. Tris-Puffer | 50 mMol |
| 4. bidest. Wasser ad. | 1 l (pH = 7,5 mit HCl) |

Die Probe wird gemischt und 20 Sekunden inkubiert. Danach werden 500 $\mu$l eines 50 mMol Tris-Puffers mit einem pH-Wert von 7,5 zugegeben. Es wird gerührt und anschließend werden je 50 $\mu$l der Farbstofflösungen A und B zugegeben:

| Lösung A: | |
|---|---|
| 1. Safranilin | 50 mg |
| 2. Tris-Puffer | 50 mMol |
| 3. Natriumchlorid | 100 mMol |
| 4. bidest. Wasser ad. | 1 l (pH = 7,5 mit HCl) |
| Lösung B: | |
| 1. Thioflavin TCN | 100 mg |
| 2. Tris-Puffer | 50 mMol |
| 3. Natriumchlorid | 100 mMol |
| 4. bidest. Wasser ad. | 1 l (pH = 7,5 mit HCl) |

Es wird wiederum gerührt und die Probe dem Durchflußcytometer zugeführt. Die Zellen werden mit einer Wellenlänge von 488 nm bestrahlt und die dabei hervorgerufene Fluoreszenz im Bereich zwischen 500 und 550 sowie im Bereich zwischen 570 und 630 nm gemessen. Ebenso wird die Intensität des Vorwärtsstreulichtes und des Weitwinkelstreulichtes (90°) , das beim Durchfließen der Zellen auftritt, aufgenommen. Mit den Signalen des Vorwärtsstreulichtes und/oder des 90°-Streulichtes werden die anderen drei Signale getriggert, d.h. nur wenn beim Vorwärtsstreulicht eine gewisse Signalintensität überschritten wird, werden auch die Werte für 90°-Streulicht sowie für die Fluoreszenz I und Fluoreszenz II aufgenommen.

Die Korrelation von Fluoreszenz I und Fluoreszenz II ergibt ein zweidimensionales Histogramm, das aufgenommen wird. In x-Richtung wird die Signalintensität der Fluoreszenz I, in y-Richtung die Signalintensität der Fluoreszenz II aufgetragen, während in z-Richtung die Anzahl der Zellen mit der jeweiligen Kombination der Signalintensität mit Fluoreszenz I und Fluoreszenz II dargestellt wird. Abbildung 3 zeigt das erhaltene zweidimensionale Histogramm mit den dazugehörigen eindimensionalen Histogrammen.

Wie der Abbildung zu entnehmen ist, dominieren zwei Bereiche das Histogramm; ein Bereich, der Zellen repräsentiert mit einer hohen Fluoreszenz-I- und Fluoreszenz-II-Intensität und ein zweiter Bereich mit einer geringen Intensität von Fluoreszenz I und Fluoreszenz II. Die Zuordnung der biologisch relevanten Zellpopulationen zu den jeweiligen Clustern im zweidimensionalen Histogramm ist der Abbildung 3 zu entnehmen.

Beispiel 2

Ein Tropfen venösen EDTA-Bluts wird auf einen Mikroskop-Objektträger gegeben und mit einem Tropfen Vorbehandlungsreagenz versetzt.
Das Vorbehandlungsreagenz weist folgende Zusammensetzung auf:

| 1. Saponin | 0,75 g |
|---|---|
| 2. Formaldehyd (37 %) | 5 ml |
| 3. Dinatriumhydrogenphosphat | 0,3 g |
| 4. Natriumdihydrogenphosphat | 0,45 g |
| 5. bidest. Wasser ad. | 100 ml |

Nach 10sekündigem Warten wird diese Mischung mit einem Tropfen Färbereagenz versetzt, das folgende Zusammensetzung hat:

| 1. Thioflavin TCN | 250 mg |
|---|---|
| 2. Safranilin | 250 mg |
| 3. Tris-Puffer | 50 mMol |
| 4. bidest. Wasser ad. | 1 l |

Die Lösung wird auf einen pH-wert von 7,5 eingestellt.
In diesem Beispiel zeigen die gefärbten suspendierten Zellen die gleichen optischen Eigenschaften bei Bestrahlung mit blauem Licht und bei Beobachtung der Emission oberhalb 500 nm im Fluoreszenzmikroskop, wie sie bereits oben als charaktieristische Merkmale der verschiedenen Leukozyten beschrieben wurden.
Analoge Ergebnisse erhält man, wenn anstelle von Thioflavin TCN und Safranilin Primulin und Basic Red 8 eingesetzt werden.

Beispiel 3

Korrelation von Daten, die am Flowcytometer gewonnen wurden, mit den bei entsprechenden Versuchen am Mikroskop gewonnenen Daten

EDTA-Blut von 250 Probanden wird jeweils in Analogie zu den Beispielen 1 und 2 am Mikroskop manuell differenziert und am Durchflußcytometer vermessen und aufgrund der angegebenen Populationsgrenzen die Menge an Lymphozyten, Monozyten, neutrophilen, eosinophilen und basophilen Granula bestimmt. Die Daten wurden nach der Methode von Bablok-Pasing korreliert. Die entsprechenden statistischen Werte zeigen eine eindeutige Korrelation zwischen den Vergleichsmethoden.

Beispiel 4

Vermessung isolierter Lymphozytenpopulationen am Durchflußcytometer

Aus EDTA-Vollblut werden mit Hilfe der Elutriation Lymphozyten mit einem Reinheitsgrad > 95 % isoliert. Die Reinheit der Lymphozytenpopulationen wird durch Antikörper-Färbung mit einem PAN-Lymphozyten-Antikörper nachgewiesen. Nach Isolation werden 50 $\mu$l Zellsuspension (ca. 5 x 10^5 Zellen/ml) mit 50 $\mu$l Vorbehandlungsreagenz versetzt, das die folgende Zusammensetzung hat:

| 1. Digitonin | 5 g |
|---|---|
| 2. Formalin (37 %) | 75 ml |
| 3. Tris-Puffer | 100 mMol |
| 4. bidest. Wasser ad. | 1 l (pH = 8,0) |

Nach 10sekündiger Inkubation wird die Suspension mit 500 μl Tris-Puffer, pH 7,5, versetzt. Nach einer weiteren 10sekündigen Inkubation werden 50 μl einer Farbreagenzlösung folgender Zusammensetzung zugegeben:

| 1. Safranilin | 500 mg |
|---|---|
| 2. Thioflavin TCN | 500 mg |
| 3. Tris-Puffer | 100 mMol |
| 4. Natriumchlorid | 20 mMol |
| 5. bidest. Wasser ad. | 1 l (pH = 7,5 mit HCl) |

Nach Mischung der beiden Lösungen wird die gefärbte Lymphozytenpopulation dem Durchflußcytometer zugeführt. Es werden analog Beispiel 1 Vorwärtsstreulicht, 90°-Streulicht, Fluoreszenz I und Fluoreszenz II ge messen. Im zweidimensionalen Histogramm, in dem Fluoreszenz I gegen Fluoreszenz II aufgetragen ist, ist lediglich eine einzige Population von Zellen wiederzufinden. Die relative Lage im Histogramm ist identisch mit den bereits vorher als Lymphozyten beschriebenen Populationen.

Beispiel 5

Sortierung eosinophiler Granulozyten

Entsprechend Beispiel 2 werden 50 μl Zellen vorbehandelt, gefärbt und dem Durchflußcytometer zur Vermessung zugeführt. Nach Triggerung über das Vorwärtsstreulicht werden die Fluoreszenz I und die Fluoreszenz II der Zellen vermessen. Der den eosinophilen Granulozyten zugehörige Cluster-Bereich wird markiert. Danach können die Zellen, die in derart markierte Bereiche fallen, vom Rest der Zellen aussortiert werden. Die so gewonnenen Zellen werden auf einen Objektträger gebracht, ausgestrichen und luftgetrocknet. Um die verbliebenen Farbstoffreste von der flowcytometrischen Färbung zu entfernen, werden die Zellen weiterbehandelt. Zunächst werden sie eine Minute mit dest. Wasser, danach mit Natriumacetat-Puffer, pH 5, 5 Minuten lang behandelt. Im Anschluß daran folgt eine Giemsa-Färbung mit 1 : 10 verdünnter Giemsa-Stammlösung.

Als optimal erweist sich eine Färbezeit von ca. 8-10 Minuten. Die gefärbten Präparate werden durch Eintauchen in der aufsteigenden Alkoholreihe von 25, 50, 70, 80, 90 und 100 % für jeweils 15 Sekunden entwässert. Anschließend werden die Objektträger für 30 Minuten in Xylol überführt und dann eingedeckt.

Die durch Sortierung gewonnenen gefärbten Objektträger werden mit einem Fluoreszenzmikroskop betrachtet, und im Anschluß daran werden die Zellen fotografiert. Es wird eine einheitliche Population eosinophiler Zellen gewonnen, die durch keine anderen Zelltypen verunreinigt ist.

## Ansprüche

1. Verfahren zur Differenzierung und quantitativen Bestimmung von Leukozyten in Vollblut mit Hilfe der Durchflußcytometrie durch Messung von Zellvolumen, Streulicht und Fluoreszenz, dadurch gekennzeichnet, daß man das Vollblut mit einem die Erythrozyten lysierenden und die Leukozyten fixierenden Agens behandelt, mit einem Gemisch von mindestens zwei Fluoreszenzfarbstoffen aus der Thiazol- und Aminoxanthengruppe versetzt, die gefärbten Zellen mit einer Wellenlänge anregt und die relativen Intensitäten der Fluoreszenzemission bei mindestens zwei verschiedenen Wellenlängenbereichen gleichzeitig mißt und das Vorwärts- und Weitwinkelstreulicht bestimmt.

2. Mittel zur Durchführung des Verfahrens nach Anspruch 1, enthaltend eine lysierend und fixierend wirkendes Agens und ein Farbreagenz.

3. Mittel nach Anspruch 2, dadurch gekennzeichnet, daß das lysierend und fixierend wirkende Agens im wesentlichen ein Saponin und einen niederen Aldehyd und das Farbreagenz mindestens je einen Farbstoff aus der Thiazol- und Aminoxanthengruppe enthält.

4. Verwendung des Mittels nach den Ansprüchen 2 und 3 zur Differenzierung und quantitativen Bestimmung von Leukozyten in Vollblut in der Durchflußcytometrie oder der Mikroskopie.

Abb. 1

Abb. 2

Abb. 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | WO-A-8 505 640 (COULTER ELECTRONICS INC.) <br> * Seite 3, Zeilen 14-35; Seite 5, Zeilen 18-19 * <br> --- | 1-4 | G 01 N 33/50 <br> G 01 N 15/14 |
| Y | EP-A-0 008 428 (BOEHRINGER MANNHEIM GmbH) <br> * Seite 4, Zeilen 25-28; Seite 9, Zeile 15 * <br> --- | 1-4 | |
| Y | EP-A-0 259 834 (TOA MEDICAL ELECTRONICS CO.) <br> * Seite 5, Zeilen 1-4; Seite 15, Zeilen 1-10 * <br> --- | 1-4 | |
| X | BIOLOGICAL ABSTRACTS, Band 83, Nr. 4, 1987, Zusammenfassung Nr. 30462, Biological Abstracts, Inc., Philadelphia, US; L.G. LEE et al.: "Thiazole orange: A new dye for reticulocyte analysis", CYTOMETRY, 7(6): 508-517 1986 <br> * Zusammenfassung * <br> ----- | 3,4 | RECHERCHIERTE SACHGEBIETE (Int. Cl.4) <br><br> G 01 N |

Der vorliegende Recherchenbericht wurde für alle Patentansprüche erstellt

| Recherchenort | Abschlußdatum der Recherche | Prüfer |
|---|---|---|
| DEN HAAG | 24-08-1989 | VAN BOHEMEN C.G. |